# EUROPEAN PATENT APPLICATION

(11) **EP 3 480 295 A1**
(43) Date of publication of application: **08.05.2019**
(21) Application number: 17200389.9
(22) Date of filing: 07.11.2017
(51) Int. Cl.: C12N 1/14, C12R 1/645, C12N 15/01, C02F 3/34, C12P 13/04, C12P 21/02, C12P 19/04, C02F 101/16

(54) **MICRO-ORGANISM TOLERANT FOR INORGANIC NITROGEN COMPOUNDS**

(71) Applicant: BioSolum B.V., 6001 AC Weert (NL)
(72) Inventor: SIEMERINK, Marco Antonius Josef, 6001 AC Weert (NL); KUIT, Wouter, 6942TD Didam (NL)
(74) Representative: Wittop Koning, Tom Hugo

(57) **Abstract**

Described is a novel micro-organism, having a nitrite tolerance of at least 200 mg/l and a nitrite clearance rate of at least 100 mg/l/d nitrite, as well as a method for the preparation of a monoculture of a micro-organism having such nitrite tolerance and clearance rate. Further, methods for clearing of inorganic nitrogen and for the preparation of one or more organic nitrogen containing compounds from a liquid source comprising inorganic nitrogen as presented.

## Description

The invention relates to a novel nitrite tolerant organism, capable of using one or more inorganic nitrogen compounds as nitrogen source, while removing said one or more inorganic nitrogen compounds from a liquid source.

The term 'inorganic nitrogen compounds' as used herein, is intended to encompass at least ammonia, nitrite and nitrate or a combination of two or more thereof, unless otherwise indicated. Such inorganic nitrogen compounds are undesired compounds in waste/fertilizer streams, in particular in waste streams, originating from intensive livestock (of i.e. pigs, cattle, poultry), where high amounts of dung are produced. Said dung comprises high levels of inorganic nitrogen compounds, such as ammonium. Stables where such intensive livestock is placed, have a too high level of the odorous ammonia in the air, so that the air must be cleaned before being discharged to the environment. Such removal of ammonia usually takes place by so-called air scrubbers (e.g. biological and/or chemical), wherein a liquid is brought in contact with the polluted air and as a result, the ammonia dissolves in the liquid. In chemical air washers, the liquid is processed with sulphuric acid, which reacts with the ammonia to form ammonium sulphate. Nevertheless, sulphuric acid is a potential treat for the environment. In biological air washers, bacteria are present that are responsible for oxidation of ammonia into nitrite followed by further oxidation of the nitrite to nitrate. For this process, called nitrification, different species are known, e.g. *Nitrosomas* and *Nitrobacter.* The problem with the biological air scrubbers is that not all of the ammonia is converted to nitrate, resulting in the inorganic nitrogen compounds ammonia, nitrite and nitrate in the residual water.

Another example where elevated levels of inorganic nitrogen compounds are generated, is in breeding water of aquatic animal breeding, e.g. shrimps and fish. Such polluted breeding water is also often treated by addition of a cocktail of *Nitrosomas* and *Nitrobacter* species. WO2006/044499 describes a consortium of *Nitrosomas eutropha* and *Nitrobacter winogradskyi,* capable of nitrification. Said consortium may work for small pollution as observed for shrimp breeding water, but is not suitable for removal of nitrite from aqueous liquids that are produced by the above described air scrubbers (connected to reversed osmosis), due to the high concentrations of inorganic nitrogen compounds therein, where nitrite concentrations higher than 10 gram per liter nitrite are observed. Such concentrations are toxic to the micro-organisms, presently known and used for removal of inorganic nitrogen compounds from liquid aqueous media. Therefore, there is a need to provide a micro-organism that is tolerant for one or more inorganic nitrogen compounds at such high concentrations, that is also capable of removing said one or more inorganic nitrogen compounds, and preferably also of nitrification and/or nitrogen assimilation into organic nitrogen containing compounds.

The present inventors have now identified a hitherto unknown micro-organism deposited at the Westerdijk Fungal Biodiversity Institute (previously known as Centraalbureau voor Schimmelcultures (CBS-KNAW)) under accession number CBS143205, that is tolerant for at least nitrite and ammonia and capable of nitrite and ammonia removal (also at higher concentrations of nitrite and ammonia in the medium). Further, said micro-organism is capable of using ammonia, nitrite and nitrate as nitrogen source and assimilation of said inorganic nitrogen compounds into organic nitrogen containing compounds. The tolerance is preferably for nitrite and/or ammonia, in particular for nitrite.

The micro-organism of the present disclosure preferably has an increased tolerance for inorganic nitrogen compounds, and has a nitrite tolerance of at least 200 mg/l, more preferably of at least 500 mg/l, even more preferably of 1 g/l nitrite, even more preferably of at least 2 g/l nitrite, even more preferably of at least 3.5 g/l nitrite, even more preferably of at least 5 g/l nitrite, even more preferably of at least 7 g/l nitrite, and most preferably of at least 10 g/l or even 12 g/l nitrite at a cultivation pH of 6.0. This means that the micro-organism can survive and utilize the inorganic nitrogen present (as nitrate, nitrite of ammonium) at such levels of nitrite at pH 6.0.

The micro-organism of the present disclosure preferably have a nitrite clearance rate of at least 100, more preferably at least 250, more preferably of at least 500 and most preferably of at least 630 mg/l/d, at a pH of 6.0. The starting concentration of nitrite is preferably 12.6 g/l. This means that the organism was capable to remove nitrite as nitrogen source in 250 ml shake flasks with 100 ml medium (biological air scrubber water supplemented with 200 mM phosphate and 30 g/l glucose; pH 6.0) at gentle agitation of 200 rpm and 20°C.

Further described is a method for the preparation of a monoculture of a micro-organism as described above, i.e. having a nitrite tolerance of at least 200 mg/l, more preferably of at least 500 mg/l, even more preferably of 1 g/l nitrite, even more preferably of at least 2 g/l nitrite, even more preferably of at least 3.5 g/l nitrite, even more preferably of at least 3.9 g/l nitrite, even more preferably of at least 5 g/l nitrite, even more preferably of at least 7 g/l nitrite, and most preferably of at least 10 g/l or even 12 g/l nitrite at a cultivation pH of 6.0, and a nitrite clearance rate at least 100, more preferably at least 250, more preferably of at least 500 and most preferably of at least 630 mg/l/d, at a pH of 6.0, comprising the steps of:
A) providing an aqueous medium having a nitrite concentration of at least 200mg/l,
B) supplementing the aqueous medium of step A) with an organic carbon source, to a carbon level corresponding with that of at least 1 g/l glucose and a phosphate source to a level of at least 1 mM.
C) allowing micro-organisms to grow,
D) inoculating micro-organisms from step C) into a fresh aqueous medium comprising a similar level of organic carbon source and phosphate as the aqueous medium after step B) and allow the micro-organisms to grow,
E) plating micro-organisms from step D) on a solid medium and allowing growth of single colonies thereon,
F) inoculating single colony micro-organisms from step E) into a fresh aqueous medium comprising a similar level of organic carbon source and phosphate as the aqueous medium after step B) and allow the micro-organisms to grow,
G) optionally repeat steps E) and F) until a monoculture of the micro-organism is obtained.

The aqueous medium in step A) can e.g. be residual water from a biological air scrubber as described above, or any aqueous medium containing inorganic nitrogen compounds that need to be removed therefrom or need to be assimilated into organic nitrogen containing compounds. Used breeding water of aquatic animals or industrial residual water containing inorganic nitrogen compounds can also be used in step A). The nitrite concentration corresponds preferably with the envisaged nitrite tolerance of the micro-organism to be identified, and is preferably 5 g/l, more preferably 10 g/l, and most preferably 12.6 g/l. It is also possible to replace nitrite in step A) for another inorganic nitrogen compound such as ammonia, in order to prepare a monoculture of a micro-organism that is tolerant for the said inorganic nitrogen compound.

In step B) the aqueous water is supplemented with a carbon and a phosphorous source. The carbon source can e.g. be glucose, whereas the phosphor source can e.g be a phosphate such as Na₂HPO₄. The amount of carbon is chosen such that it corresponds with the carbon provided by at least 1 g/l glucose, but preferably with 10 g/l glucose or even 30 g/l glucose. It has been observed that the amount of carbon needed seems to be depend on the amount of nitrogen, which needs to be removed.

The amount of phosphor is chosen such that it corresponds with at least 1 mM Na₂HPO₄, preferably at least 5 mM, more preferably at least 10 mM, more preferably at least 20 mM, more preferably at least 50 mM, more preferably at least 100 mM, most preferably at least 200mM Na₂HPO₄.

Subsequently, the micro-organisms are allowed to grow in step C), in particular for 3 weeks at 30°C and a pH typically around 6, preferably under mild agitation, such as at 200 rpm.

In step D), the micro-organisms of step C) are brought into an aqueous medium that has similar levels of carbon source and phosphate as the levels obtained in step B). For example, an inoculum of e.g.2 ml is taken from step C). Preferably, the aqueous medium in step D), is identical to that of step B). So if in step B) air scrubber residual water, supplemented with glucose and phosphate is used, the same medium is preferably used in step D). However, in step D), it is also possible to use a different aqueous medium, such as a growth medium comprising similar or preferably identical amounts of carbon source and phosphate. Thereafter, the micro-organism is allowed to grow again.

Subsequently, the micro-organisms are allowed to grow on a solid growth medium in the form of single colonies. If a fungus is envisaged to be isolated, the medium is suitable for fungal growth, such as 2% PDA (Potato Dextrose Agar such as potato extract glucose broth manufactured by Carl Roth, Germany supplemented with 2% agar), comprising an antibiotic to prevent bacterial growth such as chloramphenicol at a concentration of e.g. 34 µg/ml. However, this method is also suitable to identify other micro-organisms than fungi, such as bacteria, and to this end, the growth medium can be made suitable for bacterial growth.

If necessary for obtaining a monoculture, the steps of inoculating and growing can be repeated until a significant fast growing micro-organism, in particular a fungus is able to grow on high concentration of inorganic nitrogen compounds in the medium, in particular nitrite. This way, the deposited micro-organism as described above has been found.

For growth of the micro-organism in steps B), D) and F), the organic carbon source is preferably supplemented to a carbon level corresponding with that of at least 10 g/l glucose. The phosphate source is preferably supplemented to a level of at least 200mM.

Preferably, the carbon source comprises glucose, although also other carbon sources known to the skilled person can be used.

By the above-described method, a micro-organism can be obtained having a nitrite tolerance of at least 200 mg/l nitrite and/or a nitrite clearance rate of at least 100 mg/l/d nitrite, in particular of the preferred values as described above.

As will be further exemplified in the following examples, the micro-organism as described above preferably is a fungus belonging to the family of the *Trichocomaceae.*

Also disclosed is a method for clearing of inorganic nitrogen from a liquid source comprising inorganic nitrogen, comprising the steps of
a) contacting micro-organisms as described above with the liquid source,
b) allowing the micro-organisms to grow in the liquid source, providing the liquid source cleared from inorganic nitrogen.

By contacting the described micro-organisms with a liquid source comprising inorganic nitrogen, said inorganic nitrogen will be subjected to biological growth, resulting in clearing of nitrite present in the liquid. This was done by inoculation of the fungus into medium containing air scrubber water enriched with glucose and phosphate and grow for 20 days at 30°C and a pH of 6.0. It was found that the nitrite level of the liquid source could be brought from an initial level of 12,6 g/l nitrite to 0 g/l after 20 days at 30°C and a pH of 6.0, optionally at mild agitation, such as at 200 rpm.

Also disclosed is a method for the preparation of one or more organic nitrogen containing compounds from a liquid source comprising inorganic nitrogen, comprising the steps of
a) contacting micro-organisms as described above with the liquid source,
b) allowing the micro-organisms to grow in the liquid source,
c) purifying the one or more organic nitrogen containing compounds from the liquid source of step b).

The biomass of the fungi is grown as described above. The biomass of fungi comprises chitin. Due to the fact that glucose and nitrogen are consumed while biomass is formed, it is most likely that the carbon and nitrogen are used for biomass assimilation, such as chitin.

The one or more organic nitrogen containing compounds preferably are or comprise amino acids, such as in particular commercial interesting enzymes. Other nitrogen containing compounds, such as chitin can be prepared and purified as well.

The following examples are given to further illustrate the present disclosure.

### Example 1

### Preparation and identification of the novel micro-organism

The micro-organism of the present disclosure was identified in 250 ml shake flasks with 100 ml medium (biological air scrubber water supplemented with 200 mM phosphate and 10 g/l glucose; pH 6.0) at 200 rpm and 20°C. The micro-organisms were allowed to grow for 3 weeks at 20°C and a pH typically around 6. After 3 weeks, an inoculum of 2 ml was taken and brought into refreshed identical medium, based on residual air scrubber water, supplemented with glucose and phosphate, where after the micro-organism was allowed to grow again. These steps of inoculating and growing were repeated until a significant fast growing fungi was able to grow on high concentration of nitrite in the medium.

The nuclear rRNA cistron has been used for fungal diagnostics and phylogenetics for more than 20 year (2016 Begerow). Preliminary Next-Generation sequencing reveal at least 18 rRNAs and 70 tRNAs in the novel micro-organism, which are provided in SEQ ID No 1 - 88. The rRNAs and tRNAs sequences were identified with Barrnap and Aragorn. Barrnap is a software program that predicts the location of ribosomal RNA genes in genomes. It supports bacteria (5S,23S,16S), archaea (5S,5.8S,23S,16S), mitochondria (12S,16S) and eukaryotes (5S,5.8S,28S,18S). ARAGORN is a program commonly used to detect tRNA genes in nucleotide sequences (http://mbio-serv2.mbioekol.lu.se/ARAGORN/).
The eukaryotic rRNA cistron consists of the 18S, 5.8S, and 28S rRNA genes transcribed as a unit by RNA polymerase I. Posttranscriptional processes split the cistron, removing two internal transcribed spacers. These two spacers, including the 5.8S gene, are usually referred to as the ITS region. The 18S nuclear ribosomal small subunit rRNA gene (SSU) and the 28S nuclear ribosomal large subunit rRNA gene (LSU) sometimes discriminates species on its own or combined with ITS. LSU, a favored phylogenetic marker among many mycologists, had virtually no amplification, sequencing, alignment, or editing problems, and the barcode gap was superior to the SSU. However, across the fungal kingdom, ITS was generally superior to LSU in species discrimination and had a more clearly defined barcode gap (2016 Begerow).
The ITS RNA sequence (643 nt) of this unidentified fungi was determined by PCR and has been designated SEQ ID No 89. Internet nucleotide blast search (https://blast.ncbi.nlm.nih.gov/Blast.cgi?PROGRAM=blastn&PAGE TYPE=BlastSear ch&LINK LOC=blasthome; Database Nucleotide collection (nr/nt) using Megablast (Optimize for highly similar sequences) reveals an identity of only 91% with *Phialosimplex chlamydosporus,* suggesting a new genus and species.

Next to ITS, LSU and SSU, protein-coding genes are also widely used in mycology for phylogenetic analyses or species identification. For Ascomycota (including mold genera such as Aspergillus), they are generally superior to rRNA genes for resolving relationships at various taxonomic levels. Among protein-coding genes, the largest subunit of RNA polymerase II (RPB1) may have potential as a fungal barcode; together with two additional optional genes, namely the second largest subunit of RNA polymerase II (RPB2) and a gene encoding a minichromosome maintenance protein (MCM7).

The LSU, SSU, RPB1, RPB2, MCM7 were extracted from Next-Generation sequencing data and were designated SEQ ID No 90, 91, 92, 93, 94, respectively. LSU was found with the primers LR0R 5'-ACCCGCTGAACTTAAGC-3'; forward and LR5 5'-TCCTGAGGGAAACTTCG-3'; reverse and for SSU with the primers NS1 5'-GTAGTCATATGCTTGTCTC-3'; forward and NS4 5'-CTTCCGTCAATTCCTTTAAG-3'; reverse (http://www.fungalbarcoding.org/Defaultlnfo.aspx?Page=Primers). The protein encoding sequences RPB1, RPB2, MCM7 were found in the annotated preliminary next generation sequence data.

Internet nucleotide blast search (https://blast.ncbi.nlm.nih.qov/Blast.cgi?PROGRAM=blastn&PAGE TYPE=BlastSear ch&LINK LOC=blasthome; Database Nucleotide collection (nr/nt) using Megablast (Optimize for highly similar sequences) of SEQ ID No 90, 91, 92, 93, 94 revealed an identity of only 97%, 94%, 78%(1^{st} part)/75% (2^{nd} part), 80%, 78% with *Sagenomella sp. HMH-2007a* 28S ribosomal RNA gene, partial sequence; *Trichocoma paradoxa* gene for 18S rRNA, partial sequence, strain:IFO 6765; *Aspergillus niger* contig An01c0390, genomic *contig*/*Aspergillus terreus NIH2624* conserved hypothetical protein (ATEG_00681) partial mRNA; *Aspergillus niger* contig An12c0030, genomic contig; *Aspergillus fischeri* NRRL 181 DNA replication licensing factor Mcm7, putative partial mRNA, respectively.

Therefore, the invention also encompasses a micro-organism having an ITS RNA sequence having at least 97%, preferably at least 98, or 99% sequence identity with SEQ ID NO 89. The tRNA and rRNA of said micro-organism preferably has a sequence identity with SEQ ID Nos 1 - 88 (with a query coverage of 100%) of at least 95%, more preferably of at least 98%, or 99%. Said micro-organism preferably has a sequence identity of at least 97%, preferably at least 98% or more preferably of at least 99% with SEQ ID NO 90-94.

### Example 2

Clearing of inorganic nitrogen from a liquid inorganic nitrogen source The micro-organism of the present disclosure was inoculated in 250 mL shake flasks with 100 mL medium (biological air scrubber water supplemented with 200 mM phosphate and 10 g/L glucose; pH 6,0) at 200 rpm and 20°C. The micro-organisms were allowed to grow for 19 days at 30°C and a pH of 6.

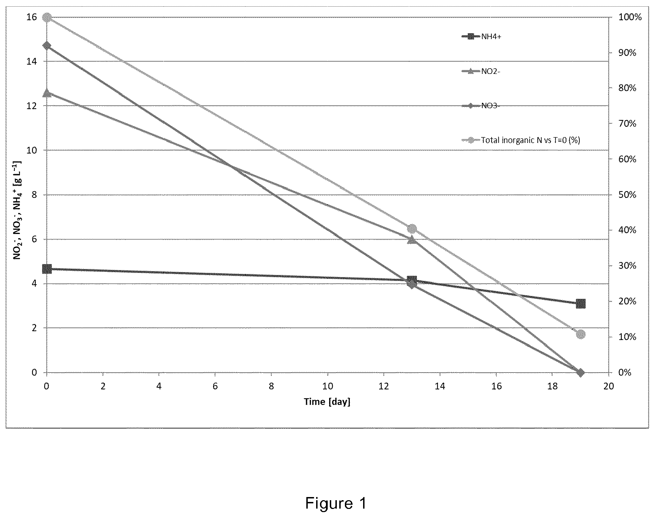

The concentrations of the inorganic nitrogen compounds of the biological air scrubber water are depicted in table 1 and figure 1, wherein concentration of nitrite, nitrate, and ammonium of biological air scrubber water are depicted in time.

Concentration of inorganic compounds in g/l in (concentrated) biological air scrubber water in time

| | 0 days | 13 days | 19 days |
|---|---|---|---|
| NH₄⁺ | 4.7 | 4.1 | 3.1 |
| NO₂⁻ | 12.6 | 6.0 | 0.0 |
| NO₃⁻ | 65.1 | 17.5 | 0.0 |

## Claims

1. Micro-organism, deposited at the Westerdijk Fungal Biodiversity Institute under accession number CBS143205.

2. Micro-organism having a having an ITS RNA sequence having at least 97%, preferably at least 98, most preferably at least 99% sequence identity with SEQ ID NO 89.

3. Micro-organism of claim 2, having at least 97% sequence identity with SEQ ID NO 90-94.

4. Micro-organism of claim 2 or 3, having at least 95% sequence identity, preferably of at least 98%, more preferably of at least 99% with SEQ ID Nos 1 - 88.

5. Micro-organism of any of the preceding claims, having a nitrite tolerance of at least 200 mg/l nitrite at a pH of 6.0.

6. Micro-organism of any of the preceding claims, having a nitrite tolerance of at least 500 mg/l, preferably of 1 g/l nitrite, more preferably of at least 2 g/l nitrite, even more preferably of at least 3.5 g/l nitrite, even more preferably of at least 5 g/l nitrite, even more preferably of at least 7 g/l nitrite, even more preferably of at least 10 g/l and most preferably of at least 12 g/l nitrite at a cultivation pH of 6.0.

7. Micro-organism of any of the preceding claims, having a nitrite clearance rate of at least 100 mg/l/d nitrite at a pH of 6.0.

8. Micro-organism of any of the preceding claims, having a nitrite clearance rate of at least 250, preferably of at least 500 and most preferably of at least 630 mg/l/d, at a pH of 6.0.

9. Method for the preparation of a monoculture of a micro-organism having a nitrite tolerance of at least 200 mg/l nitrite and a nitrite clearance rate of at least 100 mg/l/d nitrite at a pH of 6,0, comprising the steps of:
A) providing an aqueous medium having a nitrite concentration of at least 200 mg/l,
B) supplementing the aqueous medium of step A) with an organic carbon source, to a carbon level corresponding with that of at least 1 g/l glucose and a phosphate source to a level of at least 20mM,
C) allowing micro-organisms to grow,
D) inoculating micro-organisms from step C) into a fresh aqueous medium comprising a similar level of organic carbon source and phosphate as the aqueous medium after step B) and allow the micro-organisms to grow,
E) plating micro-organisms from step D) on a solid medium and allowing growth of single colonies thereon,
F) inoculating single colony micro-organisms from step E) into a fresh aqueous medium comprising a similar level of organic carbon source and phosphate as the aqueous medium after step B) and allow the micro-organisms to grow,
G) optionally repeat steps E) and F) until a monoculture of the micro-organism is obtained.

10. Method of claim of claim 9, wherein in step B) the organic carbon source is supplemented to a carbon level corresponding with that of at least 10 g/l glucose and/or wherein the phosphate source is supplemented to a level of at least 200mM.

11. Method of claim 9 or 10, wherein the carbon source comprises glucose.

12. Micro-organism, obtainable by the method of any of claims 9 - 11, having a nitrite tolerance of at least 10 g/l nitrite and/or a nitrite clearance rate of at least 500 mg/l/d nitrite.

13. Micro-organism of any of the claims 1 - 8 or 12, being a fungus belonging to the family of the *Trichocomaceae.*

14. Method for clearing of inorganic nitrogen from a liquid source comprising inorganic nitrogen comprising the steps of
a) contacting micro-organisms of any of the claims 1 - 8, 12 or 13 with the liquid source,
b) allowing the micro-organisms to grow in the liquid source, providing the liquid source cleared from inorganic nitrogen.

15. Method for the preparation of one or more organic nitrogen containing compounds from a liquid source comprising inorganic nitrogen, comprising the steps of
a) contacting micro-organisms of any of the claims 1 - 8, 12 or 13 with the liquid source,
b) allowing the micro-organisms to grow in the liquid source,
c) purifying the one or more organic nitrogen containing compounds from the liquid source of step b).


SEQUENCE LISTING

<110>  Biosolum B.V.

<120>  Micro-organism tolerant for inorganic nitrogen compounds

<130>  70367EP

<160>  94

<170>  PatentIn version 3.5

<210>  1
<211>  115
<212>  DNA
<213>  Unknown

<220>
<223>  newly identified micro-organism

<400>  1
tacgaccata gggtgtggaa aacagggctt cccgtccgct cagccgtact taagccacac      60

gccggccggt tagtagttgg gtgggtgacc accagcgaat cccggctgtt gtatg          115


<210>  2
<211>  115
<212>  DNA
<213>  Unknown

<220>
<223>  Newly identified micro-organism

<400>  2
atacaacagc cgggattcgc tggtggtcac ccacccaact actaaccggc cggcgtgtgg      60

cttaagtacg gctgagcgga cgggaagccc tgttttccac accctatggt cgtat          115


<210>  3
<211>  112
<212>  DNA
<213>  Unknown

<220>
<223>  Newly identified micro-organism

<400>  3
acgaccatag ggtgtggaaa acagggcttc ccgtccgctc agccgtactt aagccacacg      60

ccggccggtt agtagttggg tgggtgacca ccagcgaatc ccggctgttg ta             112


<210>  4
<211>  1280
<212>  DNA
<213>  Unknown

<220>
<223>  Newly identified micro-organism

<400>  4

tcctcctttc ggcgcttgac gagttgttag taccaaactg aaggaaaatt caccgtgacg 60

atggtgattc acgattacta gtaattcctt attcatgtag tcgagttaca gactacaatc 120

catattatat ccatttttgg ggattagctc aatttcgcaa tgtcgcatcc ctttgttatg 180

gcgtatgtgg cacgtctgta gcccacagta tttaggccat gatgacttgt cttaatccct 240

attacatacc tagataatgg tgaaccactt tatgtatgta aataaagtga ggggtttcgt 300

taattatagg taattaccaa atctcacgac atcaactaaa aacagccatg cagcacttgt 360

aatatttatt caaactgtgg taaggttttt cgcggatcat cgaattaaac aacatacttc 420

attactgatc tcagtaacgg tctaatgatt tcagtttata tgttgccata ttactcttga 480

ggtggaatgc ttacactttc atttataggc taaatttatt atctatctag cctatggcac 540

tcatcattgt ctgcttggac tactagggta tctaatccta attgataccc aagccttcgt 600

ccctcaacgt cagtttttac atagaaggat gccttcgccg ttaccagtct ctccggtatt 660

aaaatatgca atcactactc cagaaattct tccttctctt ataaaactct agtaaaaaaa 720

cactgtttta gtattttatt taccgtctag gtacccttta aacctaataa agatgaataa 780

cactagtctt atacgtttta ccgcgactgc tggcacgtat tttggtcaag acttataaat 840

agaatagttg tcattatctc tattctattt agaattttat acgaattaat cgttattgta 900

ttattataca ttttcattct tccaagttac tggttcagcc tttcggccat tgaccaatat 960

tcctcactgc tgtacaaaaa cgcattgggg ttttttcaga cccaatgtgg tcgatcgacc 1020

tctcagtcac gactacggat attagctaga ataagtaatt acctttccta ctactcaccg 1080

tgataaaaat taacatctta aagcagacct tatattcctt tcttttataa gggatcttat 1140

ccccttactt taaggtagcc aatttatctt tactcacctg tacaccacct acttattagt 1200

tttttttact aattagtcgt tcgattagca tgtgtcaagt tcttggacag cgttcaatca 1260

gagccatcat caagctcaac 1280

<210> 5
<211> 2103
<212> DNA
<213> Unknown

<220>
<223> Newly identified micro-organism

<400> 5
ctaaagtaag cttcgcgctt aaaatgcttt cagcacttat ctttaactga tgtagctttc 60

ctgccatgcc tatgaaaaca atttatttaa gtattaataa accatatatt aataattaat 120

taatatataa cataaacaac aggtaaacca tagatcaaca tacccaactc ctttcgtacg 180

aaggggctat ctttattata ctttaatttc ctctagaaga tagaaaccat actgtctcac 240

gacgtattaa acccagctca tgtaactctt taatcgacga acagtcgaac ccttcatgat 300

ttttgcattc atgtggatga gttaagccga catcgaggtg ccaaaccgcg cactcaattt 360

gtactctctt gcgcagatta gcctgttcaa ttttttgtta ttctaaattt ccatttctct 420

caaaatggtt cagactattt catcatcttt actaactatt tttaagcaaa aaataaataa 480

taattaacta ccacttaatc aacctttaat tccaaaagat ccactacgaa atttagaatt 540

taaattagta tagtttaaat taggttttaa gttacctctt aatactgagg gattataacc 600

catcatataa gaatatacat tatctagatt tcctttatat tttaattttt gttgagattt 660

tgaagcagaa ctacgtttag ttaatctacc tgcagcttct actctaacac ctgatactct 720

tttatattta atattagata atacaatttt tttcagatat tttgaacttg ttttattttg 780

ttctattaaa tcatttaata aagtatttgt aatatcctta tcattatcat ttacagtaaa 840

taaactatct aaatcaaaaa aatattttgg tctttttatt ttaatatttt taatttttac 900

tttatcgact aaaattgtaa gatattttaa tactttagtt atttttcttt ttcttaattt 960

taatattaaa gattctgtaa atatatcact attaaagaaa aaatatttta aattaataat 1020

attaaactct actttttttt tatatatttt ttttattaaa ctaattaaac cttgtaaata 1080

taaattatca aatttagctt tattaatata aagtaattgt ttaaaataca tgtaatattt 1140

taatcttata agtgatttat ttataaaata tttataataa agattttgta ctaaagatac 1200

ttttgaacta taattaggta aaacatttat tattatttta cctttttttt cttgttgttt 1260

tataatattc aatcctacat ttcttattaa ttttaatttt tttaataatt tacgattttt 1320

aaataatctt ttatattttt ttactatttt taataagtaa tttaatcttt gtctattata 1380

tatatataat gttatattta ctttatcatt agtatgttta aattcaccat cactaacaaa 1440

tattttattt atagataatt ttcttaatct atgacgtaat atttttattt tttttcttaa 1500

tttatattct atatttaaat tatatgaatt taaataacct ttaattaatt taagtacaaa 1560

tctgctagca ataggtatta aatttaaagc atttttatta taaacataaa tactattatt 1620

tcaatctctt acagcaggaa caaaatcttt attataaata gtaacttttg gattatttaa 1680

tgattttttt ttatatgtat tttttaattt tgattttata atatttaaca tatttatata 1740

taatattaaa gtttagctaa aatagctaaa tctatttcat tattataatt aaagtaataa 1800

taattattat acaaaattat tatagttagt aaaaatgttg ggttaattat taaagtctag 1860

tcgttgaacg gctttatttg ctacaataaa atccgcttca aattcacaga ggggaaaccc 1920

tcagttgtct ttgaaattta cccaattaca ccaatatttt acaatattgg gggactaaca 1980

tccctagcgt aactttttca aaaatccaag accattcctt aatgcatctt gtgatcatat 2040

gccccgctta cgcgactgat agatttgtag atcaaacagt aaagcaagat ttagccatta 2100

aac 2103

<210>  6
<211>  492
<212>  DNA
<213>  Unknown

<220>
<223>  Newly identified micro-organism

<400>  6
cagtaaagct gcatagggtc ttctcgtcta tctagaggta aacagtatct gcactgctat      60

tccaatttca ctgagacaat catcgagaca gctgttgtat cgttacatca ttcatgcagg     120

accgcattta acggccaagg tatttcgcta ccttaggacc ctcatagata aggccgccat     180

taattggggt atccttcaaa acctaagcta aaaacctagg taaatccgtt atccttccaa     240

cattagggca gatatcacac tcaatacttt gatattttat ctttgcagag tgctgtgttt     300

ttattaaaca gtcgtacaac attatttcat gtttcctctt ttcattaatc taatttaatg     360

ctttaaattt atgaaaatgg ccctccttat tccaaagtta cggagtcaat ttgccgagtt     420

ccttaatgat tgttctctca aacgcctttg tattctctac ttgcttactt gtgatagtat     480

ctaggtacgg tt                                                         492


<210>  7
<211>  747
<212>  DNA
<213>  Unknown

<220>
<223>  Newly identified micro-organism

<400>  7
aaagtttcgg tatattgttt agatccgtta aattttcggt gtttttttct aagtttaatc      60

agtgctctgt tacgaggtct tcaaaaaatg gccgcttcta agcctatttc ctgattgttt     120

aaaaaaaaaa gcatccttaa tttcacttaa caataatttt ggaaccttaa cctttgttct     180

gggctgtttc cctttagaca tacaacctta tcgcaatatg tccgattatt caacatacat     240

ctttgccatt tcgagtcgat cactctccga taaagtcact acaacccccc gatataaaca     300

aaatattgtt cgagatcaca gttctgtacc tgctaagatg ttaattaaat tacctactta     360

tataggtttc gcggaaaacc agctatacta gtcagttttg tctttcacta acttaccaca     420

attcttcgga tatctataca acgataaccc gttcggactt ttataatcct gaccatggta     480

agatcactag ctttcgggtc taattctaga tactcattca ttttttcata attggtttat     540

ctaagctagt ttaatacatt aaacatcgct tgcatctaaa attaacttgc tgacccatta     600

tgcaaaaggt acgctcttat tatttactta aatttaacta aagctgctta taaaactact     660

atttcaaatt ttacctcacg gcactttttc actatcgctt atatactata tttagcctta     720

gaggaaggtt cccctttatt caaacag                                         747

<210>  8
<211>  115
<212>  DNA
<213>  Unknown

<220>
<223>  Newly identified micro-organism

<400>  8
tacgaccata gggtgtggaa aacagggctt cccgtccgct cagccgtact taagccacac      60

gccggccggt tagtagttgg gtgggtgacc accagcgaat cccggctgtt gtatg          115


<210>  9
<211>  115
<212>  DNA
<213>  Unknown

<220>
<223>  Newly identified micro-organism

<400>  9
atacaacagc cgggattcgc tggtggtcac ccacccaact actaaccggc cggcgtgtgg      60

cttaagtacg gctgagcgga cgggaagccc tgttttccac accctatggt cgtat          115


<210>  10
<211>  115
<212>  DNA
<213>  Unknown

<220>
<223>  Newly identified micro-organism

<400>  10
tacgaccata gggtgtggag aacagggctt cccgtccgct cagccgtact taagccacac      60

gccggccggt tagtagttgg gtgggtgacc accagcgaat cccggctgtt gtatg          115


<210>  11
<211>  115
<212>  DNA
<213>  Unknown

<220>
<223>  Newly identified micro-organism

<400>  11
tacgaccata gggtgtggaa agcagggctt cccgtccgct cagccgtact taagccacac      60

gccggccggt tagtagttgg gtgggtgacc accagcgaat cccggctgtt gtatg          115


<210>  12
<211>  115
<212>  DNA
<213>  Unknown

<220>
<223>  Newly identified micro-organism

<400>  12
tacgaccata gggtgtggaa agcagggctt cccgtccgct cagccgtact taagccacac      60

gccggccggt tagtagttgg gtgggtgacc accagcgaat cccggctgtt gtatg          115


<210>  13
<211>  115
<212>  DNA
<213>  Unknown

<220>
<223>  Newly identified micro-organism

<400>  13
atacaacagc cgggattcgc tggtggtcac ccacccaact actaaccggc cggcgtgtgg      60

cttaagtacg gctgagcgga cgggaagccc tgttttccac accctatggt cgtat          115


<210>  14
<211>  443
<212>  DNA
<213>  Unknown

<220>
<223>  Newly identified micro-organism

<400>  14
tctggttgat tctgccagta gtcatatgct tgtctcaaag attaagccat gcatgtctaa      60

gtataagcac tttatacggt gaaactgcga atggctcatt aaatcagtta tcgtttattt     120

gatagtgcct tactacatgg atacctgtgg taattctaga gctaatacat gctgaaaacc     180

ccgacttcgg aaggggtgta tttattagat aaaaaaccaa cgcccttcgg ggctccttgg     240

tgaatcatga taacttaacg aatcgcatgg ccttgcgccg gcgatggttc attcaaattt     300

ctgccctatc aactttcgat ggtaggatag tggcctacca tggtggcaac gggtaacggg     360

gaattagggt tcgattccgg agagggagcc tgagaaacgg ctaccacatc caaggaaggc     420

agcaggcgcg caaattaccc aat                                             443


<210>  15
<211>  296
<212>  DNA
<213>  Unknown

<220>
<223>  Newly identified micro-organism

<400>  15
agtcgggggc gtcagtattc agctgtcaga ggtgaaattc ttggatttgc tgaagactaa      60

ctactgcgaa agcattcgcc aaggatgttt tcattaatca gggaacgaaa gttaggggat     120

cgaagacgat cagataccgt cgtagtctta accataaact atgccgacta gggatcggac     180

ggtgtttcta tgatgacccg ttcggcacct tacgagaaat caaagttttt gggttctggg 240

gggagtatgg tcgcaaggct gaaacttaaa gaaattgacg gaagggcacc acaagg 296

<210> 16
<211> 638
<212> DNA
<213> Unknown

<220>
<223> Newly identified micro-organism

<400> 16
cacgggagcc tgcggcttaa tttgactcaa cacggggaaa ctcaccaggt ccagacaaaa 60

taaggattga cagattgaga gctctttctt gatcttttgg atggtggtgc atggccgttc 120

ttagttggtg gagtgatttg tctgcttaat tgcgataacg aacgagacct cggcccttaa 180

atagcccgac ccgcgttcgc gggccgctgg cttcttaggg ggactatcgg ctcaagccga 240

tggaagtgcg cggcaataac aggtctgtga tgcccttaga tgttctgggc cgcacgcgcg 300

ctacactgac agggccagcg agtacatcac cttggccgag aggcccgggt aatcttgtta 360

aaccctgtcg tgctggggat agagcattgc aattattgct cttcaacgag gaatgcctag 420

taggcacgag tcatcagctc gtgccgatta cgtccctgcc ctttgtacac accgcccgtc 480

gctactaccg attgaatggc tcggtgaggc cttcggactg gcccaggggg gttggaaacg 540

accccccagg gccggaaagt tggtcaaacc cggtcattta gaggaagtaa aagtcgtaac 600

aaggtttccg taggtgaacc tgcggaagga tcattacc 638

<210> 17
<211> 4960
<212> DNA
<213> Unknown

<220>
<223> Newly identified micro-organism

<400> 17
aaaactttca acaacggatc tcttggttcc ggcatcgatg aagaacgcag cgaactgcga 60

taagtagtgt gaattgcaga attcagtgaa tcatcgagtc tttgaacgca cattgcgccc 120

cctggcattc cggggggcat gcctgtccga gcgtcattgc taccctcaag cgcggcttgt 180

gtgttgggcc gtcgtccccc ccggtgcttc cggcggggga cgggcccgaa aggcagtggc 240

ggcaccgcga ccggtcctcg agcgtatggg gctctgtcac ccgctccgtg ggaccggccg 300

gcgccagccc tccaaatatg tatatatata tatatacata ccaagttgac ctcggatcag 360

gtagggatac ccgctgaact taagcatatc aataagcgga ggaaaagaaa ccaaccggga 420

ttgccctagt aacggcgagt gaagcggcaa gagctcaaat ttgaaatctg gcccctccgg 480

ggtccgagtt gtaatttgta gaggatgctt cgggtgcggc ccccgtcgaa gtgccctgga 540

acgggccgtc ggagagggtg agaatcccgt ctgggatggg gtgcccgcgc ccgtgtgaag 600

ctccctcgac gagtcgagtt gtttgggaat gcagctctaa gtgggtggta aatttcatct 660

aaagctaaat accggccgga gaccgatagc gcacaagtag agtgatcgaa agatgaaaag 720

caccttgaaa agggagtgaa acagcacgtg aaattgttga aagggaagcg ctggcgacca 780

gactcgcccg cggggttcag ccggcattcg tgccggtgta cttccccgtg ggcgggccag 840

cgtcggtttg ggcggccggt caaaggcccc cggaatgtag cgcctctcgg ggcgccttat 900

agccgggggt gcaatgcggc cagcccggac cgaggaacgc gcttcggcac ggacgctggc 960

ataatggtcg tcaacgaccc gtcttgaaac acggaccaag gagtctaaca tctacgcgag 1020

tgctcgggtg gcaaacccgt gcgcgcagtg aaagcgaacg gaggtaggag cccccccgtg 1080

gggcgcacta tcgaccgatc ctgatgtctt cggatggatt tgagtaggag cgtagctgtg 1140

gggacccgaa agatggtgaa ctatgcctga atagggtgaa gccaggggaa actctggtgg 1200

aggctcgcag cggttctgac gtgcaaatcg atcgtcaaat ttgggtatag gggcgaaaga 1260

ctaatcgaac catctagtag ctggttcctg ccgaagtttc cctcaggata gcagtaacgc 1320

ggatcagttt tatgaggtaa agcgaatgat tagaggcctt ggggttgaaa caaccttaac 1380

ctattctcaa actttaaata tgtaagaagc ccttgtttct tcagtgaacg cgggcattcg 1440

aatggtgcgt tactagtggg ccatttttgg taagcagaac tggcgatgcg ggatgaaccg 1500

aacgcgaggt taaggtgccg gaatgcacgc tcatcagaca ccacaaaagg tgttagttca 1560

tctagacagc ccgacggtgg ccatggaagt cggaatccgc taaggagtgt gtaacaactc 1620

acgggccgaa tgaactagcc ctgaaaatgg atggcgctca agcgtgctac cgatacctcg 1680

ccgccggggt agaagcgacg ccccggcgag taggcaggcg tgggggtccg tgacgaagcc 1740

cagggagtga tcccgggtcg aacggcccct agtgcagatc ttggtggtag tagcaaatac 1800

tcaaatgaga actttgagga ctgaagtggg gaaaggttcc atgtgaacag caattggaca 1860

tgggttagtc gatcctaagg catagggaag ttccgtttga aaggcgccct gcgcgccgtg 1920

tgccgaaagg gaagccggtt aacattccgg cacctggatg tggattctcc acggcaacgt 1980

aactgaacgc ggagacgtcg gcgggggccc tgggaagagt tctcttttct tcttgacggc 2040

ctatcaccct gaaatcggtt tgtccggagc tagggttcaa cggccggcag agccccgcac 2100

ctttgcgggg tccggtgcgc ccccgacgac ccttgaaaat ccgcgggaag gaatagtttt 2160

cacgccaggt cgtactcata accgcagcag gtctccaagg tgaacagcct ctagttgata 2220

gaacaatgta gataagggaa gtcggcaaaa tggatccgta acttcgggat aaggattggc 2280

tctaagggtc gggcccgctg ggccttgggg ggaacccccc ggagcagggg ggcactagcc 2340

gggcaaccgg ccggcgcccc ccagcaccgg gcgggggacg cccttggcag gcttcggccg 2400

tccggcgggc gcttaacgac caacttagaa ctggtacgga caaggggaat ctgactgtct 2460
aattaaaaca tagcattgcg atggccagaa agtggtgttg acgcaatgtg atttctgccc 2520
agtgctctga atgtcaaagt gaagaaattc aaccaagcgc gggtaaacgg cgggagtaac 2580
tatgactcga gaatcacgtg gacgaacgat ggctaccaca gtgagagggt caagcggcgt 2640
gaggagaaga cgtcgctagt gaggggggga ggagcacgga ggggcggtcc cggcccggcg 2700
tgcctcgtgt ccccttgcga caccgtcaaa ttgcgggaac ttcctaaagc ctctgccacc 2760
gcggctcgcc gaaaggcgca gtgcagcacc agggtaatga cctcggggat ggtaatagcg 2820
cagaggatgt gccaatggat gatccgcagc caaaccctac gtcccccccg ctccaggggg 2880
ggatacgggc gcagtccaac gactagatgg cggtgggtct cccggggaag gccccggggg 2940
gtctaagata tagtctgacg gtccgccgaa aggcgtgtgt ggccgcagaa tgctcttaag 3000
gtagccaaat gcctcgtcat ctaattagtg acgcgcatga atggattaac gagattccca 3060
ctgtccctat ctactatcta gcgaaaccac agccaaggga acgggcttgg caaaatcagc 3120
ggggaaagaa gaccctgttg agcttgactc tagtttgaca ttgtgaaaag acatatgggg 3180
tgtagaatag gtgggagctt cggcgccggt gaaataccac tacctttatc gtttttttac 3240
ttattcaatg aagcggaact gggcttcacc gcccatcttc tggcgttaag gtccttcgcg 3300
ggccgatccg ggttgaagac attgtcaggt ggggagtttg gctggggcgg cacatctgtt 3360
aaaccacaac gcaggtgtcc taagggggac tcatcgcgaa cagaaatcgc gagtggagca 3420
aaagggcaaa agtccccttg attttgattt tcagtgtgaa tacaaaccat gaaagtgtgg 3480
cctatcgatc ctttatgtcc ctcgaaattt gaggctagag gtgccagaaa agttaccaca 3540
gggattaact agatatagtc ccaggccccg acgcctggtt ggggtctagt ccggctgccg 3600
tcgagactgt gcgggtggca gctaagaaag tcccggtcgt ccgggccttt ctgtagggca 3660
acacatctgg atgcggggaa ctctcgccag gtctctagta ccaggtggcc gggtaacgcc 3720
cggtcacgga gtctgttcag ggcagacggg taaaaaccta gggaatagag acaatccgca 3780
gccgacccgg ctccctgccc cccttccgtc ggggcaccag ctgggcggtt caacgctcgg 3840
taagatgtgg gtgcgccccc cctggggggc gcgcttaagg tacgggctac gcccacccga 3900
gagggtgcgg ccgcctaccg accggcatag ggtcgagaag cggccggtgc gggctcaccg 3960
gtgatgcgtc ctgcctgccc cccttgtccg ggggggccag gaccccggag ggcccgctgg 4020
tagtacggaa ctggcttgtg gcagccaagc gttcatagcg acgttgcttt ttgatccttc 4080
gatgtcggct cttcctatca taccgaagca gaattcggta agcgttggat tgttcaccca 4140
ctaaaccatc gagaacgtat agttccccat tggtggggat agccgggcct ccgcgagggc 4200
gcgggggtta ggccggctag tcgatacagc aggaccgctg caggtgctgt acggcgacac 4260

aacctggatc gggggaggct gccgggggcc acggagcgcc gtggtcgcca tgctaatccc 4320

gagccgagcc gcggtggagc gatccgccgt cgaggccggt gtagagcgcg gaaaggtgtg 4380

ggtggcgctg ggggtcagac ctcaggccgc ttaagggacg tgccaacccc tagggaaacc 4440

tagcccgagt gcgaaggtcc tgcaggccgg atcacacggg gggcagtgag ccgcggagga 4500

aatgccgtgg ggagcctggt atgggcgggg gaggtggatc gacacccccc cgaccccatc 4560

gtgaataggg aacgtgagct gggtttagac cgtcgtgaga caggttagtt ttaccctact 4620

gatgaaggtc gccgcaacgg taattcaatt tagtacgaga ggaaccgttg attcagataa 4680

ttggtttttg cggctgtctg atcaggcagt gccgcgacgc taccatctgc cggataatgg 4740

ctgaacgcct ctaagtcaga atccgtgccg gaacgcggcg atatcgcccc gcacgtcgta 4800

gttggatacg aataggcctc cgggccatgg acctcagcag gctggcgacg gttcccgggg 4860

agaagccccc gggagccagc tggcggattg caatgtcacc tcgcgcgggg atagatcctc 4920

tgcagacgac tgaagtgacc aagcgggtcg tgtaagcgct 4960

<210> 18
<211> 115
<212> DNA
<213> Unknown

<220>
<223> Newly identified micro-organism

<400> 18
tacgaccata gggtgtggaa aacagggctt cccgtccgct cagccgtact taagccacac 60

gccggccggt tagtagttgg gtgggtgacc accagcgaat cccggctgtt gtatg 115

<210> 19
<211> 84
<212> DNA
<213> Unknown

<220>
<223> Newly identified micro-organism

<400> 19
cttctcgatg gtccagtggt caagataccc gcttgtcatt cgacaagccg cgggtgaccg 60

gggttcgatt ccccgtcggg aagt 84

<210> 20
<211> 72
<212> DNA
<213> Unknown

<220>
<223> Newly identified micro-organism

<400> 20
tccaatgtgg tctaacggct aggataccga gctttcacct cggaggtcgg ggttcgattc 60

cccgcattgg aa                                                          72


<210>  21
<211>  83
<212>  DNA
<213>  Unknown

<220>
<223>  Newly identified micro-organism

<400>  21
tggcaagatg gccgagcggt taaggcgcat gtttcaggta catgttctgc aaagatcgtg      60

ggttcgaatc ccactcttgt cat                                              83


<210>  22
<211>  83
<212>  DNA
<213>  Unknown

<220>
<223>  Newly identified micro-organism

<400>  22
gacctgctgg tgtagtggtt atcacgcaat gctcacatga attgtgccat tgaggtgctc      60

ggttcgatcc cgaggtaggt cac                                              83


<210>  23
<211>  74
<212>  DNA
<213>  Unknown

<220>
<223>  Newly identified micro-organism

<400>  23
taggtgatta gtttagtggc agaacgtgtc gtttacaccg acaaggtcga cggttcgatc      60

ccgtcatcac ctaa                                                        74


<210>  24
<211>  74
<212>  DNA
<213>  Unknown

<220>
<223>  Newly identified micro-organism

<400>  24
agtcctttag gatagctggt tattccgcat cgttaacacc gatgaggtcg gcggttcgaa      60

cccgtcaagg acta                                                        74


<210>  25
<211>  74
<212>  DNA

<213>  Unknown

<220>
<223>  Newly identified micro-organism

<400>  25
agtcctttag gatagctggt tattccgcat cgttaacacc gatgaggtcg gcggttcgaa      60

cccgtcaagg acta                                                        74


<210>  26
<211>  88
<212>  DNA
<213>  Unknown

<220>
<223>  Newly identified micro-organism

<400>  26
gggcttgtgg tctagtggta tgacgcgtca ttcgcatatt ctgcaatgga aattgatgag      60

gtccagggtt caattccctg caagtcca                                         88


<210>  27
<211>  81
<212>  DNA
<213>  Unknown

<220>
<223>  Newly identified micro-organism

<400>  27
ggccggctgg cctaatggtt aaggcttcac tctcctactc tggttaagtg aggattgcgg      60

gttcgaatcc cgtgtcggtc g                                                81


<210>  28
<211>  72
<212>  DNA
<213>  Unknown

<220>
<223>  Newly identified micro-organism

<400>  28
gcatcattgg ttcagtggta gaattcctga cttccaatca gatgacccgc gttcgattcg      60

cggatgatgc ac                                                          72


<210>  29
<211>  83
<212>  DNA
<213>  Unknown

<220>
<223>  Newly identified micro-organism

<400>  29
tggcaggatg gccgagcggt taaggcgcag cattagaaat gctgttcttt cgagatcgtg      60

ggttcgaatc ccactcctgt cga                                          83

<210>  30
<211>  83
<212>  DNA
<213>  Unknown

<220>
<223>  Newly identified micro-organism

<400>  30
tggcgagatg gccgagcggt taaggcgcta gagtaaggtt ctagtatcga aagatgcatg      60

ggttcgaatc ccattctcgt cat                                          83

<210>  31
<211>  72
<212>  DNA
<213>  Unknown

<220>
<223>  Newly identified micro-organism

<400>  31
agcatggtag ctcaggggaa gagcgccggg ctcataaccc ggaggtccct ggatcgaaac      60

caggccatgc ta                                                      72

<210>  32
<211>  81
<212>  DNA
<213>  Unknown

<220>
<223>  Newly identified micro-organism

<400>  32
ggcgagatgg ccgagcggtt aaggcgctag agtaaggttc tagtatcgaa aggtgcatgg      60

gttcgaatcc cattctcgtc a                                            81

<210>  33
<211>  72
<212>  DNA
<213>  Unknown

<220>
<223>  Newly identified micro-organism

<400>  33
tccggtatgg tgtagtggtt aacatcgccg tctctcacac ggcagccggg ggttcgattc      60

cccctaccgg aa                                                      72

<210>  34
<211>  88
<212>  DNA

<213>  Unknown

<220>
<223>  Newly identified micro-organism

<400>  34
agccctcgtg gcctaactgg ttaaggcgta aggcttctat ttattaaggc gaccttaaga      60

ttgcgggttc gagtcccgcc gggggttt                                          88


<210>  35
<211>  72
<212>  DNA
<213>  Unknown

<220>
<223>  Newly identified micro-organism

<400>  35
tccggtatgg tgtagtggtt aacatcgccg tctctcacac ggcagccggg ggttcgattc      60

cccctaccgg aa                                                           72


<210>  36
<211>  81
<212>  DNA
<213>  Unknown

<220>
<223>  Newly identified micro-organism

<400>  36
ttctcgatgg tccagtggtc aagatacccg cttgtcattg acaaatcgcg ggtgaccggg      60

gttcgattcc ccgtcgggaa g                                                 81


<210>  37
<211>  87
<212>  DNA
<213>  Unknown

<220>
<223>  Newly identified micro-organism

<400>  37
ggtcctatgg tctaggggtt aggacgtcgg attttgatta ttatttccga acttccgaaa      60

gcgagggttc gaatccctct aggacct                                           87


<210>  38
<211>  71
<212>  DNA
<213>  Unknown

<220>
<223>  Newly identified micro-organism

<400>  38
gcccgcttag tctagtggta agacgtttca ctcgtaatga aaagtggtca gttcgattct      60

ggcagtgggc a                                                          71


<210>  39
<211>  75
<212>  DNA
<213>  Unknown

<220>
<223>  Newly identified micro-organism

<400>  39
aggtcgcgtg gctcaattgg cagagtgctt gactacgaat caagaggttc ggggttcgaa      60

cccccgcgtg acctt                                                      75


<210>  40
<211>  71
<212>  DNA
<213>  Unknown

<220>
<223>  Newly identified micro-organism

<400>  40
gcattgttgg tctagtggta gaattcatcg ttgccatcga tgaggcccgc gttcgattcg      60

cggacaatgc a                                                          71


<210>  41
<211>  71
<212>  DNA
<213>  Unknown

<220>
<223>  Newly identified micro-organism

<400>  41
gcattgttgg tctagtggta gaattcatcg ttgccatcga tgaggcccgc gttcgattcg      60

cggacaatgc a                                                          71


<210>  42
<211>  75
<212>  DNA
<213>  Unknown

<220>
<223>  Newly identified micro-organism

<400>  42
tgcattgttg gtctagtggt agaattcatc gttgccatcg atgaggcccg cgttcgattc      60

gcggacaatg catcc                                                      75


<210>  43
<211>  71
<212>  DNA

<213>  Unknown

<220>
<223>  Newly identified micro-organism

<400>  43
gcattgttgg tctagtggta gaattcatcg ttgccatcga tgaggcccgc gttcgattcg      60

cggacaatgc a                                                           71


<210>  44
<211>  104
<212>  DNA
<213>  Unknown

<220>
<223>  Newly identified micro-organism

<400>  44
tgactttgct ggcgtagtgg acgcgacatc ttcgtgactc gagttcacgg atggcgatat      60

agattgccgg gtgttattct gttcctctct cggagaaggg tcgg                      104


<210>  45
<211>  87
<212>  DNA
<213>  Unknown

<220>
<223>  Newly identified micro-organism

<400>  45
ggtcctgtag ctcaatggtg agagtttcaa gcttatgggc atggttacat tccttgacgg      60

ttgtgggttc gagtcccacc gggacca                                          87


<210>  46
<211>  90
<212>  DNA
<213>  Unknown

<220>
<223>  Newly identified micro-organism

<400>  46
ggaagcctgg atgaggagcg ccctagaatc tgttggattc tatgttgagc acatagagac      60

agagtatcgt cccacgcgac tgacctccac                                       90


<210>  47
<211>  81
<212>  DNA
<213>  Unknown

<220>
<223>  Newly identified micro-organism

<400>  47
tgcattgatg gtgtagtggt atcacaccga ccttccatac cgagagtcgg taaccggggt      60

tcgattcccc gtcaacgcat c                                                81

<210>  48
<211>  73
<212>  DNA
<213>  Unknown

<220>
<223>  Newly identified micro-organism

<400>  48
gtcctcgtgg cccagcggtt aaggcgctcg gctgttaacc gagagatcgg aggttcgatc      60

cctcccgtgg acg                                                         73

<210>  49
<211>  81
<212>  DNA
<213>  Unknown

<220>
<223>  Newly identified micro-organism

<400>  49
aggttgtatg gtctagtggt tatgacgtgt gactctgatg tggaatcaca tagccttggt      60

tcgattccga gtacgaccta c                                                81

<210>  50
<211>  73
<212>  DNA
<213>  Unknown

<220>
<223>  Newly identified micro-organism

<400>  50
ggtcgtatgg tctagtggtt atgacgtgtg actctgaatc acatagcctt ggttcgattc      60

cgagtacgac ctc                                                         73

<210>  51
<211>  85
<212>  DNA
<213>  Unknown

<220>
<223>  Newly identified micro-organism

<400>  51
gcgccgatgg tcaagtggtt acgatgtggt cctcccattc tgtgaggtta gaccgagacc      60

cgagttcgaa tcccggtcgg cgcac                                            85

<210>  52
<211>  75
<212>  DNA

<213>  Unknown

<220>
<223>  Newly identified micro-organism

<400>  52
tggccgtgtg gcgcaatggt agcgcgtcgg tttccggtac cgaaggcttg cgggttcgaa      60

tcccgtcatg gtcga                                                      75


<210>  53
<211>  72
<212>  DNA
<213>  Unknown

<220>
<223>  Newly identified micro-organism

<400>  53
ggtgctatag tctaacggtc aggacggcac gttgtggccg tgcaggtttg ggttcgattc      60

ccaatggcgc ca                                                         72


<210>  54
<211>  85
<212>  DNA
<213>  Unknown

<220>
<223>  Newly identified micro-organism

<400>  54
aggaagggtc cgtttgttgg ttagcgggtt gagctgtaaa ctcaatggct ataggccatc      60

gaaggttcga ttccttttct tccta                                           85


<210>  55
<211>  73
<212>  DNA
<213>  Unknown

<220>
<223>  Newly identified micro-organism

<400>  55
agctcttata gctcaatggt agagcggaat actgttaata tttttataga tgttcgattc      60

atcttaaggg ctt                                                        73


<210>  56
<211>  73
<212>  DNA
<213>  Unknown

<220>
<223>  Newly identified micro-organism

<400>  56
attcttatta gctcaatggt agagcagaat acttctaata ttttgatcca agttcgagtc      60

ctggataaga ata                                                          73


<210>  57
<211>  73
<212>  DNA
<213>  Unknown

<220>
<223>  Newly identified micro-organism

<400>  57
aggcatgtta atttaatgga agaatagcgt gctacggaca cgaatatata agttcgagtc      60

ttatacatgt ctt                                                          73


<210>  58
<211>  73
<212>  DNA
<213>  Unknown

<220>
<223>  Newly identified micro-organism

<400>  58
ggaaggtata gtttaaaggt agaacagctg tatgtggcac agtaaatctt agttcgattc      60

taggtacctc cct                                                          73


<210>  59
<211>  73
<212>  DNA
<213>  Unknown

<220>
<223>  Newly identified micro-organism

<400>  59
agccagtgta gtttaatggt agaacaataa tttcatgaat taagaatgag aattcgactt      60

tctcctctgg ctt                                                          73


<210>  60
<211>  73
<212>  DNA
<213>  Unknown

<220>
<223>  Newly identified micro-organism

<400>  60
tatgttgtag actaatcggt aagtcataaa tttttggtat ttacttatgg gtgttcgagt      60

cgccccaaca tag                                                          73


<210>  61
<211>  84
<212>  DNA

<213>  Unknown

<220>
<223>  Newly identified micro-organism

<400>  61
atgggtgtgc tgaaatttgg taaccaggtt ctgcttagga cagaatagtt tttaacttta      60

caagttcgag tcttgttacc cata                                              84


<210>  62
<211>  75
<212>  DNA
<213>  Unknown

<220>
<223>  Newly identified micro-organism

<400>  62
tgcttgagaa gctcaattgg tagagcggat cactgaagat gattaggttg taagttcaag      60

tcttgtctca agcag                                                        75


<210>  63
<211>  74
<212>  DNA
<213>  Unknown

<220>
<223>  Newly identified micro-organism

<400>  63
tggggttata gtttaactgg taaaacaacg attttgcata tcgttaattc aggatcgagt      60

cctgataact ccaa                                                         74


<210>  64
<211>  82
<212>  DNA
<213>  Unknown

<220>
<223>  Newly identified micro-organism

<400>  64
atccgagtgc tggaattggt agacagtctt aacttaagat taagtggtgt aagccgtaaa      60

cgttcaagtc gtttctcgga ta                                                82


<210>  65
<211>  73
<212>  DNA
<213>  Unknown

<220>
<223>  Newly identified micro-organism

<400>  65
aaggctatag cttaataggt aaagcgaacc actcatgatg gtttgagtaa atgttcaagt      60

cattttagcc tta                                                          73


<210>  66
<211>  73
<212>  DNA
<213>  Unknown

<220>
<223>  Newly identified micro-organism

<400>  66
aagaaattag ctcagttggt agagctgtcg ctttacacgc gaaaggtcag gtgttcaagt      60

cacctatttc tta                                                          73


<210>  67
<211>  74
<212>  DNA
<213>  Unknown

<220>
<223>  Newly identified micro-organism

<400>  67
gacccaatgg tcaagtttgg ttaagacata acattttcac tgttagtgcg ggagttcaaa      60

tctcccttgg gttg                                                         74


<210>  68
<211>  75
<212>  DNA
<213>  Unknown

<220>
<223>  Newly identified micro-organism

<400>  68
tgcctttttt gcataattag ataatgcaac caccttgtaa gtggtagatg caagtgcaat      60

tcttgcatta ggcat                                                        75


<210>  69
<211>  72
<212>  DNA
<213>  Unknown

<220>
<223>  Newly identified micro-organism

<400>  69
caggttagag ccaggtggtg aggcaactta tttgggttaa gtctttttta tgttcgaatc      60

ataataacct ga                                                           72


<210>  70
<211>  86
<212>  DNA

<213>  Unknown

<220>
<223>  Newly identified micro-organism

<400>  70
agagagttgg ctgagtggtt taaagcggtt agcttgagtc taactaaagg taaaaccttt      60

catatgttcg aatcatatac tctctg                                            86


<210>  71
<211>  74
<212>  DNA
<213>  Unknown

<220>
<223>  Newly identified micro-organism

<400>  71
tgattcttta acttaatagg taaagtgtgt tcttgataag gatatgttca gtgttcgatt      60

cactgaagaa tcat                                                         74


<210>  72
<211>  72
<212>  DNA
<213>  Unknown

<220>
<223>  Newly identified micro-organism

<400>  72
aagagcatag tttaataggc aaaactgtaa gcttcaacct tatatttctt agttcaagtc      60

taagtgctct tg                                                           72


<210>  73
<211>  81
<212>  DNA
<213>  Unknown

<220>
<223>  Newly identified micro-organism

<400>  73
ggaaaagttt ccaagggtag ggtaagatat ttgctaaata ttgtgttttt acacttagat      60

gttcgattca tctcttttcc g                                                 81


<210>  74
<211>  73
<212>  DNA
<213>  Unknown

<220>
<223>  Newly identified micro-organism

<400>  74
gggttagtaa cttaataggt aaaggatttt cttgtcgaga aagtagatgc cggatcgaaa      60

ccggtctaac tcg                                                          73


<210>  75
<211>  72
<212>  DNA
<213>  Unknown

<220>
<223>  Newly identified micro-organism

<400>  75
atggctatat gtcaattggt agacatgtta tcttccacat aacaagtgtc gattcgagtt      60

cgactagcca ta                                                          72


<210>  76
<211>  71
<212>  DNA
<213>  Unknown

<220>
<223>  Newly identified micro-organism

<400>  76
atgactataa gttaacggta gactacttat ttaccaaata agatgtgctg attcgaattc      60

agctagtcat a                                                           71


<210>  77
<211>  72
<212>  DNA
<213>  Unknown

<220>
<223>  Newly identified micro-organism

<400>  77
gagactttag tttaataggc aaaaccaatg acttttaatc attttttatg ggttcaagtc      60

ccataagtct ta                                                          72


<210>  78
<211>  90
<212>  DNA
<213>  Unknown

<220>
<223>  Newly identified micro-organism

<400>  78
actgggggag tcgcgagggg aagggcggac aggtcttcgg ggcccgtgcg gatggagctg      60

atgggctggg ttctatgcct gtccccggtg                                       90


<210>  79
<211>  82
<212>  DNA

<213>  Unknown

<220>
<223>  Newly identified micro-organism

<400>  79
gggtttgtgg tccagtggta cgatgcatgc ttcgggaaga ctgatatgta tgcgacccgg      60

gttcgactcc cggcaaaccc cc                                              82


<210>  80
<211>  75
<212>  DNA
<213>  Unknown

<220>
<223>  Newly identified micro-organism

<400>  80
tgggcttgta gctcagttgg tagagcgttc gctttgcatg cgaaaagtct gggtttcgaa      60

tacccacaag tccat                                                      75


<210>  81
<211>  88
<212>  DNA
<213>  Unknown

<220>
<223>  Newly identified micro-organism

<400>  81
gcctggatag ttcagtggca gaattcatcg tttgggtata gctttgctca tctcgatgag      60

gtcctgggtt cgatccccag ttcaggcc                                        88


<210>  82
<211>  87
<212>  DNA
<213>  Unknown

<220>
<223>  Newly identified micro-organism

<400>  82
tggcaagatg gcggagtggt taacgcgttg cactcgaatg ctatgcaatc ccttcggggg      60

cctgagttcg aatctcagtc ttgtcga                                         87


<210>  83
<211>  75
<212>  DNA
<213>  Unknown

<220>
<223>  Newly identified micro-organism

<400>  83
tgtcctcgtg gcccagcggt taaggcgctc ggctgttaac cgagagatcg gaggttcgat      60

ccctcccgtg gacgt                                                    75

<210>  84
<211>  82
<212>  DNA
<213>  Unknown

<220>
<223>  Newly identified micro-organism

<400>  84
ttctcgatgg tccagtggtc aagatacccg cttgtcattc gacaagccgc gggtgaccgg      60

ggttcgattc cccgtcggga ag                                               82

<210>  85
<211>  76
<212>  DNA
<213>  Unknown

<220>
<223>  Newly identified micro-organism

<400>  85
tggtcgcgta gctcagttgg ttagagcgcg acactaataa tgtcgaggcc gtgggttcga      60

gcccctccgt gaccat                                                      76

<210>  86
<211>  76
<212>  DNA
<213>  Unknown

<220>
<223>  Newly identified micro-organism

<400>  86
tggtcgcgta gctcagttgg ttagagcgcg acactaataa tgtcgaggcc gtgggttcga      60

gcccctccgt gaccat                                                      76

<210>  87
<211>  89
<212>  DNA
<213>  Unknown

<220>
<223>  Newly identified micro-organism

<400>  87
ggaagaatgg cagagtggta atgcaccggc ttgctaagcc gagataacca gagatggctg      60

cgctggttcg ataccagctt cttccgcca                                        89

<210>  88
<211>  87
<212>  DNA

<213>  Unknown

<220>
<223>  Newly identified micro-organism

<400>  88
ggagggttct gctaggttgg ctggcaagcg gtcttgaaaa ccgtggcaac ggcaacgttg      60

ggggttcgac tcctcaaccc tccgcca                                          87


<210>  89
<211>  643
<212>  DNA
<213>  Unknown

<220>
<223>  Newly identified micro-organism

<400>  89
gtcgtaacaa ggtttccgta ggtgaacctg cggaaggatc attaccgagt gcggcacccc      60

cccgggggtg ccaacctccc acccgtgtct accgtaccat gttgcttcgg cgggcccgcc     120

cccccagcag gaggggtcgc cgggggggca tctgtccccc ccaggtgcgc gcccgccgga     180

gacacccaca cgaacgcttg tctgaaggac tgtcgtctga gtggattgat caaatcagtt     240

aaaactttca acaacggatc tcttggttcc ggcatcgatg aagaacgcag cgaactgcga     300

taagtagtgt gaattgcaga attcagtgaa tcatcgagtc tttgaacgca cattgcgccc     360

cctggcattc cggggggcat gcctgtccga gcgtcattgc taccctcaag cgcggcttgt     420

gtgttgggcc gtcgtccccc ccggtgcttc cggcggggga cgggcccgaa aggcagtggc     480

ggcaccgcga ccggtcctcg agcgtatggg gctctgtcac ccgctccgtg ggaccggccg     540

gcgccagccc tccaaatatg tatatatata tatatacata ccaagttgac ctcggatcag     600

gtagggatac ccgctgaact taagcatatc aataagcgga gga                       643


<210>  90
<211>  940
<212>  DNA
<213>  Unknown

<220>
<223>  Newly identified micro-organism

<400>  90
acccgctgaa cttaagcata tcaataagcg gaggaaaaga aaccaaccgg gattgcccta      60

gtaacggcga gtgaagcggc aagagctcaa atttgaaatc tggcccctcc ggggtccgag     120

ttgtaatttg tagaggatgc ttcgggtgcg gcccccgtcg aagtgccctg gaacgggccg     180

tcggagaggg tgagaatccc gtctgggatg gggtgcccgc gcccgtgtga agctccctcg     240

acgagtcgag ttgtttggga atgcagctct aagtgggtgg taaatttcat ctaaagctaa     300

ataccggccg gagaccgata gcgcacaagt agagtgatcg aaagatgaaa agcaccttga     360

aaagggagtg aaacagcacg tgaaattgtt gaaagggaag cgctggcgac cagactcgcc 420

cgcggggttc agccggcatt cgtgccggtg tacttccccg tgggcgggcc agcgtcggtt 480

tgggcggccg gtcaaaggcc cccggaatgt agcgcctctc ggggcgcctt atagccgggg 540

gtgcaatgcg gccagcccgg accgaggaac gcgcttcggc acggacgctg gcataatggt 600

cgtcaacgac ccgtcttgaa acacggacca aggagtctaa catctacgcg agtgctcggg 660

tggcaaaccc gtgcgcgcag tgaaagcgaa cggaggtagg agcccccccg tggggcgcac 720

tatcgaccga tcctgatgtc ttcggatgga tttgagtagg agcgtagctg tggggacccg 780

aaagatggtg aactatgcct gaatagggtg aagccagggg aaactctggt ggaggctcgc 840

agcggttctg acgtgcaaat cgatcgtcaa atttgggtat aggggcgaaa gactaatcga 900

accatctagt agctggttcc tgccgaagtt tccctcagga 940

<210> 91
<211> 1533
<212> DNA
<213> Unknown

<220>
<223> Newly identified micro-organism

<400> 91
gtagtcatat gcttgtctca aagattaagc catgcatgtc taagtataag cactttatac 60

ggtgaaactg cgaatggctc attaaatcag ttatcgttta tttgatagtg ccttactaca 120

tggatacctg tggtaattct agagctaata catgctgaaa accccgactt cggaaggggt 180

gtatttatta gataaaaaac caacgccctt cggggctcct tggtgaatca tgataactta 240

acgaatcgca tggccttgcg ccggcgatgg ttcattcaaa tttctgccct atcaactttc 300

gatggtagga tagtggccta ccatggtggc aacgggtaac ggggaattag ggttcgattc 360

cggagaggga gcctgagaaa cggctaccac atccaaggaa ggcagcaggc gcgcaaatta 420

cccaatcccg acacggggag gtagtgacaa taaatactga tacggggctc ttttgggtct 480

cgtaattgga atgagagcaa tttaaacccc tcatcgagga acaattggag ggcaagtctg 540

gtgaaccatc agttctacaa tgctgacgcc agagatagta ggaccctccc tcccggggag 600

ggttacgcct actagtcgag cactcgcaat agcgatgggt gagtgccggc gaggtgacct 660

ggtacggggg agcctaaggg tgcggcctgg aagagaggtc gacgacacct atggtaatcc 720

cgtggcgagc ctacacggcc gtcgtaacgc gcggaaaggc accggtcgct agagatctag 780

cggctcaagg gacgtgctgt cccatccgaa aggatgcctg caactggagc acccatcgtg 840

taaaggttgt ggggggtttg cagcgggccg ctgtaagcct gggggtctct catcgtggca 900

tggcgaggaa cgtcgccacg tcgatgaatg agagcgaaaa cggccagcag ccgcggtaat 960

tccagctcca atagcgtata ttaaagttgt tgcagttaaa aagctcgtag ttgaaccttg 1020

ggcctggctg gccggtccgc ctcaccgcga gtactggtcc ggctgggcct ttccttctgg 1080

ggaaccccat ggccttcact ggccgtgggg ggaaccagga cttttactgt gaaaaaatta 1140

gagtgttcaa agcaggcctt tgctcgaata cattagcatg gaataataga ataggacgtg 1200

cggttctatt ttgttggttt ctaggaccgc cgtaatgatt aatagggata gtcgggggcg 1260

tcagtattca gctgtcagag gtgaaattct tggatttgct gaagactaac tactgcgaaa 1320

gcattcgcca aggatgtttt cattaatcag ggaacgaaag ttaggggatc gaagacgatc 1380

agataccgtc gtagtcttaa ccataaacta tgccgactag ggatcggacg gtgtttctat 1440

gatgacccgt tcggcacctt acgagaaatc aaagtttttg ggttctgggg ggagtatggt 1500

cgcaaggctg aaacttaaag aaattgacgg aag 1533

<210> 92
<211> 8811
<212> DNA
<213> Unknown

<220>
<223> Newly identified micro-organism

<400> 92
atgtcgaacg tctactttcc ctactcaaaa gcgcctctgc gcaccataaa ggagatccag 60

tttggtctgt tttctcccga ggaggtgaag aggatgagcg tggtccacgt ggaatacccg 120

gagacgatgg tctgtgtcct tacgcggacc ggtcaggatg gaagatgtgc tgacaaggtc 180

aacaggacga ccagagggtg cgacctcggg ccaaaggttt gaatgatccg cgcctaggaa 240

cgatcgatcg tcagtggaac tgcgagactt gcgaggaagg ccagagagaa tgccccgggc 300

actttggcca tatcgaactg gcaaccccgg tcttccacat cggtatgccg gatactctac 360

tgtctacttg acaaattact gacactttgt ctaggctttt tgacgaaggt caaaaaactg 420

ttggagactg tctgccataa ctgtggaaag atcaaagcca acacggttag tcatccctta 480

tgggatggca aatcttatat ggcaatttgt gctgacactg gaccagtccg atccaaagtt 540

tttagaagcc atccgcatga gagatccgaa gaggcgtttc gacagcattt ggcggctctc 600

gaaagacgtt ttgatctgtg aagccgaccc atcgcctgat gatgatgaac ctttttcaaa 660

agagagctcc aaaccaatcc ggacacatgg cgggtgtggc aatgcccagc ctgtgattcg 720

caaggagggg atttccttga ccgggacatg gaagcctaat aagagcttga tggatgagat 780

ggagatacag cagcccgaaa agaaaaccat tactccgcag atggccctca atgttttccg 840

gaacatttct catgaggatg tgcgcatcat gggcttgagt aatgattacg ctcgccctga 900

gtggatgatc atcactgtcc tgccggttcc tcccccacca gttcgcccga gtgtccttgt 960

cgggggtagc tccagtggcc agcgcggtga ggatgacttg acgtacaaac ttgcagaaat 1020

tatccgtgca aatcagaacg tgcaacgctg tgagcaagaa ggtgcccctg agcacgtgat 1080

tcgcgaattc gaatctttgc tgcagtatca cgtcgccact tacatggata atgatattgc 1140

tggccagcca aaggctatgc agaagtccaa cagaccggtt aaggcgcttc gctcccgtct 1200

gaagggtaag gagggccgtc tgcggcaaaa cttgatggga aaacgtgtgg acttttccgc 1260

gcgtactgtg attacgggcg accctaatat ctccctcgac gaagtcggcg ttccgaggag 1320

cattgctagg tcactgacct atcctgaagt tgttacgcct tacaacatcg ataaattgca 1380

gatgttaatc atgaatggtc caaatgcgca tcctggagcg cgatatattg tcagagacaa 1440

tggcgaacgt atcgatttgc gtcatgcgaa aagagccggc ggtcagcagt tgctctatgg 1500

ttggaaggtc gagcgtcatc ttatggatgg tgatgtgatc ctgttcaacc ggcagccatc 1560

tctgcacaag gaatccatga tgggccaccg tgtccgtgtg atgtcatatt caacgttccg 1620

gttgaatctt tctgttacta ctccgtacaa tgctgacttc gatggcgacg agatgaacct 1680

ccacgttccg cagggcgagg aagcccgggc agagctcgat caacttgcaa tggttccttt 1740

gaacatcgtg tctccccagc ggaacggccc actcatgggt attgtgcaag acacgctttg 1800

tggtatctat aaaatttgcc gacgcgatac ctacctaacc agggaacaag tgatgaacat 1860

catgctttgg gtacctgact gggacggtgt cattcctccc ccagccatcg tgaaacctag 1920

gccaagatgg actggaaagc agatgatcag tatggctctt ccatctggcc tcaatctgct 1980

acgtgttgag aaagacagct cgactctttc tgaaaagttc tccccactaa atgatgatgg 2040

cctacttatc cacggcggac agttgatgta tgggatgttt tccaagaaaa ctgttggcgc 2100

tactggtggg ggtgtcatcc atatcatttt taatgaatat ggagcagatg ctgccgtcgc 2160

tttcttcaat ggcgcccaga ccattgtcaa ttattggctg ctgcataatg gcttcagtat 2220

cggaattggt gacacgattc cggaccctgt aacaattcag aggattgaag aatgcgttcg 2280

caatcgcaag aaggaggttg aatccattac gttaagcgca actgacaata ccctcgaagc 2340

gctgcctggt atgaacgtgc gagagacgtt tgagagtaag gtcgcgcgcg cgctcaacaa 2400

tgcacgtgat gaagctggtg gtgagaccga gaagagtttg aaggatagta accacgctat 2460

ccaaatggct cgttctggat caaagggctc gaccatcaat atttctcaga tgactgcggt 2520

cgttggacag cagtctgtgg agggcaagcg tattcccttt ggattcaagt atcgtactct 2580

accgcacttc accaaggatg actattcccc ggagtctcgt ggttttgttg aaaactctta 2640

cctgcgcgga ttgaccccta ccgaattctt tttccacgca atggctggca gggagggtct 2700

catcgatact gctgtgaaaa cggccgaaac cggttatatt cagcgaaagc ttgtcaaggc 2760

ccttgaagag gttatggtca agtacgatgg cacagttcgc aactcactgg gggatatcat 2820

ccagtttatc tacggagaag acggcctcga cggtgctcat attgagaacc agcgggtgga 2880

catcatcagg tgctctgata acaaattcat ggaccgtttc cgtatcgacg tcatggatcc 2940
tgagcggagc ctgggccctg aagtattgga acaggccaac gagattgccg gtgacatgga 3000
ggttcagcga taccttgatg aagagtggga gcaactcctc aaggaccgcg ctttccttcg 3060
ttccgtcgcc aaggaagatg aggagatgat gcagctccca atcaacgttc agaggatcct 3120
cgagtcggcc aggactactt ttaggatccg tgagggcact atcagtgatt tgcatccagc 3180
ggaagttatt cctcaagtcc aaggattact taatcgcctg ttggttgtac gcggtgatga 3240
ccctatttct catgaggcac aggaggatgc aactctactg tttaaggcac agctgcgtag 3300
ccgtcttgca ttcaagaggc tcgtcactga gtactcattg aacaagctcg cttttcagca 3360
tgtcctcggc gctattgaaa gccgatttgc gaaagctatt gccaaccctg gtgagatggt 3420
cggagtgctc tccgcacaat ccattggtga gccagctacg cagatgacac tgaacacctt 3480
ccactttgct ggtgtttcgt ccaagaacgt tacccttggt gttccacgtc tcaaagagat 3540
tctcaatgtg gctgcgaaca tcaagactcc atctatgact gtttaccagg aaccggggag 3600
agctcatgac aaagagggcg cgaagcagct gcggagcgct gtcgagcaca ccagcttaag 3660
atcagtgacg gaaagcactg agatctatta tgatccggat attcagacga ctgtgatcga 3720
aaatgacagg gacatggtag aatcttactt catcattcct gaagatgtca ccgacgattc 3780
gactcgccag tcaaagtggc tgctgcgtat cgttctcagc cgcgcgcaac tccttgacaa 3840
ggggcttacg gttcaggatg tcgcttccaa gatcaagcaa gcgtacccta gggacatcgc 3900
agtgatattc agcgacaaca acgccgatga gcaggttatc cgtattcgtc agctccagga 3960
ccataaggaa gatgatgatg atgacgagat tgaatacgat gttacactga agaaactgga 4020
ggagcacctg cttgataccc tcactcttcg cggtgttgac ggagttgagc gcgcgttcat 4080
caatgaaagg agcaaggtcc gggttctcga agatggctcc cttttctcaa gcaagagcga 4140
cccgctctgc cgagaatggg ttctcgagac tagtggctcc tcccttggcg aagttctgtc 4200
agtccctgga gtcgacgcca cccgcacgta ttccaaccag ttcattgaag tgttccacgt 4260
cttcggcgtc gaagcagctc gtgcggcggt cctccgcgaa ttgacccagg tccttgcgtt 4320
cgacggctcc tacgttaatc accgtcacct tgcacttttg gtcgacatca tgactgtacg 4380
gggctatctt acacctgtca ctcgccacgg tatcaaccgg gcagataatg gcgcacttat 4440
gcgttgttct ttcgaagaga cggtggagat tctgcttgaa gcagcggcct ttggagagct 4500
cgacgactgt cgcggtgttt cggagaattt gatcttgggc cagatggcac ctgccggtac 4560
cggagagttt gatatttacc tcgatcaggg tatgatgaac acggtcgtat cgaacaatgc 4620
gcggttcggt gtgatgggcg cgatcggcgc taaagacgcg atcatcgcgg acggtgctgc 4680
cactcagtat gacactgggt cgccgatgca ggagagtgct tatatcggca cgcccgatcc 4740
cgactcttcc ttctcgccga tcggtcaagc gggcgcggaa acccctggtg gctttacaga 4800

atatcagcct cctggattcg gaggtgggtt cagtcctgcc gccagaagcc caggtggtta 4860

ttcgcccagc agtccgttca atacgagccc gacatctcct ggatattcgc caacatcaag 4920

ctactcacct acgtcacctg gcatggggat cactagccct cgattcatgt catcgccagg 4980

cttctcaccg gctagtccgt catttgctcc aacgtctccg gcatattcgc caacatcgcc 5040

aacatcacct tcgtactccc caacatctcc agggttctca ccgacttccc ccaactacag 5100

ccctacatca ccgagcttca gtccggcgtc tcctgccttc agccccacgt cgcctagtta 5160

tagcccgacg agcccggcca tcggtggagc tggggcgaat ctgtcaccta cctccccaac 5220

atcccccaag tacacaccca cttcgccagg ctggtccccc acaagcccgc agacatactc 5280

acctacctcc cctaactttg ctggctcacc gacttcgcct ggtggaccaa cttctccagg 5340

ctacagtccg acttcgccgg cttacagtcc cacgtgagtc ccgccttatt gagctctaat 5400

gaacttctgt ggctgataat tcccgccaga tctccccgcc agtgaagtca gcacctcgta 5460

ctgtgttacg gcgaagtttc attacatcgt tgtgccacga tcggcctact gagaagaaac 5520

caaaagatac atttcgcatc ttacgttgta aaattggttg gttatttcgt tcacatcctg 5580

gccaactgca gcctattcct gatgtatcct cctctcatga accagccata gtggtttctt 5640

ctgcttctgc gcgcgccgtg tttaataggt ttccggccga ccttcgaaga ttgcccctgg 5700

ttttgggggt cggtcatgga tgttttttcg ctgaatacca agcgtcttag aactgaggct 5760

ttacaggtgg tatcgctatc gcaaggactg ctggattatt attcttattc atgtacacag 5820

ctagcagcgg tatagaaatc aagaggttgc tcgatgctcg atgatcagtg ctaggtactg 5880

tacctaaggt agtctacaca gtaaaatagt tgttacatac tgtgaagttg ggctgcgtac 5940

ggatatctgc acagaccaag tcggcaaaaa agagctccga gagacaggtg gcccattcca 6000

acttcctacg tatcttcttc aactcccccg cgggtcatgt ccgcttgaag tctatggggt 6060

cgcgatgctc gtctcactct ctacttgctt aatattctca ctggcatcaa aatgaagccg 6120

tggcactggt gctccctttc attcgcactc cgtaagtggc ccttccccac tgagctgggc 6180

ttaggttttt ttcggagaac tgatcttgca acgttctcag tgctcccgca ggtgctcgct 6240

gtccaagacc gaggtccact tgtagatgca gcacatgttt cacaccataa tgtccatcat 6300

gatgccagat catcgagtgg gaccgaagat ggtgatgttt ggcatgggaa tggtaagggt 6360

tacccggctt cgctctgaac gaacttcgct ccccccaacg gatactgata atgcaacccc 6420

ttccagaaca cgtccaaaag gctttggaaa tccttcacga ccccaagatc accgccatcc 6480

aaccaaataa gccgtctggt ttcatcggat atagcttaca ttacgcccag ttggcgtttc 6540

gcgtcctgtt cttgaacggg ccccagtccg atgcgcagga gcgaagagtc cacccgaacg 6600

tcgccaacgc tgtcaaggag ctggaaatgg ctatcgagga gagcgccaac cccgacgcga 6660

tgttcttgtt ggccgagatg aacttctatg gcaacttcac gcattcgcgc gacttcaagc 6720

aggcgtttcg atggtaccag gagcttgctt cgttgactgg aaatagtacg gcgcagtata 6780

tgatcggatt tatgtatgcg acagggatcg gcggtgcggt tgaacgcgac caagcgaagg 6840

ccttgatgta tcacactttt gccgctgagg gggggaatac aaggtcccag atgacgctcg 6900

cctatcgctt ccactccgga attgggacac cgagaaactg cgacgaggcg aactactact 6960

acaaaatggt tgcggataag gctattcggc actatcggtc aggtccgccg ggtggcaata 7020

acatggatcg ggaatcatac cgctgggttg acagagaggg tggggtgtac ggagaaggag 7080

ccagcatgtc gagttctggg ccgaatgcgc cacgagacat ctcaaacact ggcgcagagg 7140

ctagcctaga ggatatactg gagtatctgg acctcatgtc ccggaagggc gaactgaaag 7200

ctacaattag cctgggcaag atgcattacg agggggccca tggtctgccc aggaatttcc 7260

gaagggcgat gaagtatttc aagactgtca cgaggcggta ctggaacaag gatggctctg 7320

tcaatcctaa ccacccatcg ggggttgcga aactagcgtc gaaagcagcc ggccatgtcg 7380

ggacaatgta tcttcgtggt gaaggggtgg agcagaattt tggcactgcc ttaacatggt 7440

tcaaacgtgg actagccaat ggcgacgcgc tttgccaata tgaactcggc ttaatgtatc 7500

tgcatggata cggtgttcct aaagatgctt tcagagctgc ttcttacttc aaggccgcgg 7560

ctgagcagga tttcccagcg gcagagacac ggctaggcgc gctgttcctg gatcagggcg 7620

atgttgaagc ggctgcacga tatttcgagc tggctgcgcg ctggggatcg atggaggcgt 7680

tttattatct ggccgagctg tccaacggcg gagttggtag gcgacgacac tgtgggatgg 7740

caacagccta ctataagatg gttgcagagc gggctgaagc tattcattca tctttcgccg 7800

aatcgaatgc cgcctacgaa aatggcgata aagaacgagc agtcatcgct acaatgatgg 7860

cagcggagca aggatacgag aatgcgcagt ccaacgtggc ctatcttctg gatgagcaga 7920

actccttgtt ctcactcgac gcgatcctgc cttggggagg aaagtcccgg cctagtctgc 7980

ttcggaatgc ggcgcaggct ttgatctatt ggacccgttc agctaagcag gcgaatattg 8040

acgcgttgat caaaatgggc gattactacc tcgctggaat aggagctgta gcggatgcgg 8100

agaaggcatc cacctgttat cataccgccg cggaagctca tcacagtgcg caggcgtatt 8160

ggaatctcgg gtggatgcat gagaacggta tcgcggtcga gcaagatttt cacatggcca 8220

aaagatacta tgacttggca ctggaaacca accaagagtc gtatcttccg gtgaaactga 8280

gcctgataaa gctgcggatg cgaggctact ggaaccgcat caccaacggg aagatcaacc 8340

ctatccagga ggaagagagt aagtcagatc gcggcatatc atgaagagac aactgacagc 8400

ttgacagacc ccaggccaca acgaagtttc aaggaatggg tcgccgcgtt catcgaaaac 8460

gacgaggagg aggaaaatct gcgagcacat ctgtacaagc aagctaatgg ggaggacgac 8520

gatctcctct cgagcaatcg cctggagggc gaagacgagc gtgatgatgg gtactacgac 8580

gatatggacc tcgacatcga cgagagtctc ctagaggctc ttgttattct ttctctagtt 8640

gcgacgctga tgggacttct atacatgaga cagcaacgga atcgacagcg gcagaacgag 8700

gatgggggtg ctggtgcgaa tgctgcagcc gatagaggag atcgcgggtt gtttccaagg 8760

ccgggagatc cggattttgc accatgggta gctggaggag taggacatta g 8811

<210> 93
<211> 3819
<212> DNA
<213> Unknown

<220>
<223> Newly identified micro-organism

<400> 93
atggccgact acgacgaagc gtacgaggag gagttctacg aggaggagga ggaaggaatt 60

acctcggagg actgctggac ggtcatttcg tccttttttg acagcaaagg tctagtgtcg 120

cagcagctgg attccttcga tgagtttatc tcgtcgacga tgcaagagtt ggtggaggaa 180

cagggtcaag tcgtggttga tcaaactctc ccgcccgccg aagatgaagt cgatccggtg 240

gtgctccgtc gctacgaact caagtttggg acagtcatgc tctcaagacc ttcagttacg 300

gaaggcgatg gtgcgacgac tatcatgtta ccgcaggagg cccgtttgcg gaatctcacc 360

tacgccagtc cgctgtatct cgggatcacg aagagaataa tggaaggtcg cgaacgaatg 420

gtggctgatg gtgaggacgg cgatgcgggt gatttagatg agcggagagc gaaaggtaca 480

tacctccagt gggagcagaa gcagctttct gaagaccaag cgaaggagga aactgttttc 540

attggaaaga tacccattat gctcaagtct aagtactgca ttctcaagga cttgaacgag 600

caggcgctct acaattggga cgaatgcccc tatgattctg gtggctactt tatcatcaac 660

ggaagcgaga aggtcctgat cgcgcaagag cgcagcgcag ggaatatcgt ccaggttttc 720

aagaaggcgc ctccgagtcc gactccatat actgctgaaa tccgaagtgc cgtcgaaaag 780

ggttcgaggc ttctctcgca gttatctctt aaactgtttg cgaagggaga cagcgcaaaa 840

ggtggatttg gtccgacgat tagatcgacg ctaccatatg tgaaaaccga catccctatc 900

gtggtcgtgt tcagagctct cggagttgtg tccgacgagg atatcctcaa ccacatctgc 960

tacgaccgga atgatacccc tatgttggag atgctgaagc cgtgtatcga agagggcttc 1020

gtcattcaag accgtgaagt cgctcttgat ttcatcgcta agcgagggtc tacacagtca 1080

agtatgaatc acgagcggcg tgtcagatac gccagggaaa tcatgcagaa ggagttcctt 1140

ccgcatattt cgcagagcga gggaagcgag acccggaagg cgttcttctt gggatacatg 1200

gtacacagac tcttgcagtg cgccttgggt cgtcgggatg ttgatgatcg tgatcatttt 1260

ggcaagaagc gcctcgatct tgccggtccg ctactagcaa atctcttccg tgttttgttc 1320

acaagggtta cgagggacct acagcggtat gtgcagcgat gcgtggagag tggtcgagag 1380

atctacttga acattggtat caaggctagc accttgaccg gaggcctgaa atacgcgctt 1440

gcaacaggaa actggggtga gcagaagaag gccgcaagct ccaaagctgg cgtgtcgcag 1500

gtcctgagtc gttacactta cgcatctacg ctgtcacatc ttcgtcggac caacaccccc 1560

atcggtcgcg acgggaagat cgcaaagcca cggcagcttc ataacaccca ctggggcttg 1620

gtctgtccgt cagagacgcc ggaaggccag gcatgcggcc tcgtcaagaa cctggcgctc 1680

atgtgctata ttactgtcgg tacacccagc gaaccgatta tcgacttcat gatccagcgc 1740

aacatggagg tactggaaga gttcgagccg caagtaacac cgaatgcgac aaaggtgttt 1800

gtaaacggtg tctgggttgg tatccaccgc gacccgtctc accttgtgag caccatgctc 1860

tctctgcgca ggcgcaacat gatttcccac gaggtcagtt tgattcggga tatccgtgag 1920

cgagaattca aaatcttcac cgacgctggt cgcgtatcgc ggccgctgtt cgtgatagac 1980

aatgacccga agagcgagaa ttctggatct ctcgtgctca gcaaggaaca catccgaaaa 2040

ctagagcagg ataaggagct gcctccggat ctggatccag aagagcgtag ggagcgatac 2100

ttcggatggg acggccttgt gaaatcgggc gtcgtggagt atgtcgatgc ggaggaggag 2160

gaaacgatca tgatcgtgat gaccccggag gaccttgaga tcgggaaaca gcttcaggca 2220

ggatatagtc tcccggagga gatcgatcct cataagcgtg tgagatcggt tctcactcaa 2280

agagcacata cttggacgca ctgcgagatc cacccgagta tgattctggg tgtttgtgcc 2340

agtatcattc cgttcccgga tcataaccag tcgcctcgta acacttacca agcagcaatg 2400

ggcaaacaag ccatgggcgt gttcttgacc aactttgacc agcgcatgga gacgatgatg 2460

aatatccttt actatcccca gaaaccccta gcaacgacac gatcgatgga gttcctccgg 2520

ttccgagagc tgcctgccgg gcagaatgcc atcgtcgcta ttgcgtgcta ttctggatac 2580

aaccaggagg actcggttat catgaaccaa agcagtatcg accgcgggct cttccgaagc 2640

ttgttctacc ggacgtacaa cgatgctgag aagatggttg gattgacggt ggttgagcga 2700

ttcgagaggc cgatgcggac tgatacgatt ggcatgagga aaggacatac cgataagatt 2760

gacgaggatg gcatcattgc cccaggcgtg cgcgtgtccg gagaggacat catcattggc 2820

aagacggcgc ctttggcacc ggaggcggag gagctcggcc agagaaccaa agcccatacc 2880

aaactggacg tgtcaacacc actgcgaagc acggagagtg gtattgtgga taaggttctt 2940

gtttcgaccg gcagtgacga cctcaagttt gtcaaggtcc gtatgaggac gactaagatc 3000

ccccagattg gtgataagtt cgcttctcgc cacgggcaaa agggtacgat tggtgttacg 3060

taccggcagg aagacatgcc gtttactcgc gaaggcgtgg taccggacct catcatcaac 3120

cctcatgcta tcccatcgcg tatgacgatt gcccacttga ttgagtgtca gctcagcaag 3180

gtgtcggcgc tgcgagggtt cgaaggtgac gcgacgccgt ttacggatgt cactgtagat 3240

tcaatatcgc gcctgctccg cgagcacggg tatcagtcgc gtgggttcga ggtgatgtat 3300

aacggacaca cgggacgcaa gctggttgcg caggtcttcc tgggaccgac gtactaccag 3360

cggctgcgac acatggtgga tgacaagatc cacgctcgtg cacgagggcc gacacagatc 3420

ctgacacgac agccggtgga gggtagggca cgagatggtg gactacgatt cggtgagatg 3480

gagcgcgact gtatgatcgc gcatggagcc agcgcattcc tgaaggagcg gttatttgac 3540

gtatccgatc cattccgagt ccacatctgc gatgattgtg ggctaatgac gccggttgcg 3600

taagtcaacg atctgatact tcttatattg acagcacctg ggactaacta gagcagtaaa 3660

ctgaagaaag gactattcga gtgtcgactg tgcaacaaca agcaccggat ctcgcaggtc 3720

catatcccat atgccgccaa gcttctgttc caagaactgg cctcgatgaa tatcgcggca 3780

cgcatgttta ccgatcgttc tggggtgtct gtacggtag 3819

<210> 94
<211> 2589
<212> DNA
<213> Unknown

<220>
<223> Newly identified micro-organism

<400> 94
atggctctcc ttcagtaccc tgcgcccgtt gactatacgg cgcagctcaa tgctttcaag 60

gactttcttc agcacttcaa gaccttcgag tcggcatccg aatcggcggc aacggaagct 120

attgaggatc tacatatcga tggcgatggc accagcgatg aatatgattt tatggatgac 180

gtggaggatg agaacgggga gcagagacct ggtagaaggc atcgcgaacc gaagcacaag 240

tacatgcaga tgctacaaga tgtcgcagat cgggaacgga caaatgttct cattgagctg 300

gatgaccttt taattgtaag cgatcagtac tctagtgtct atcactacta actttcggac 360

gcagttcgaa agatcactct ctgaggagac aaatttgaaa ctggtcgagt ctgtgcaaag 420

aaataccaag cgctatattg atgtgttttc tcaggcggta gacgccgtca tgcccaaaga 480

gactaaggaa gtaacgtacg ctttgcccag aggaatacaa tggacgcgac taactcgcac 540

aggttcaaag atgatgtcct cgatgttatc atgtcgcaac gcgagaagcg caatgaagcc 600

atgaacttgg ccatgcagac cgatatggat gctgccgcaa ctccttcgat tttccctcct 660

gaattgaccc gtcgatacac cctcaacttt aagccggtta cactctccgg gtccagcagc 720

gaacgtgatt ctaaggcgct cgcagtccgg aatgttcgag ctgaacatct tggcagtctc 780

ataaccgtcc gtggcataac aacccgggtg tcagatgtca agccggcagt ccagataaac 840

gcctatgcat gtgatcggtg tggctgtgag gtgttccagc cgattaccac gaaacaattc 900

ctcccgctga cagagtgctt gtctgaggaa tgtatgaaga acaactccaa ggggcagctg 960

ttcctctcca cccgtgcatc gaaatttgtt ccattccaag aggtgaagat ccaggagatg 1020

gcggaccagg tccctgttgg ccatatcccg cggacattga ccatccactg tcacggaagt 1080

ctcacacggc aactcaatcc tggggactat gttgatatcg cgggaatttt ccttcctatt 1140

ccctacaccg gtttcagagc tatccgcgct ggtttgttga cggacactta catggaagcc 1200

cagcatatca ctcagcataa gaaagcatac aacgatctcg tgatggagag tcgtactctt 1260

cgaaagatcg aacagtacca aagatcgggc cagatgtatg aatacctgtc acggtccatt 1320

gctcctgaaa ttttcggtca catggatatc aagaaagccc tactactgct tctaatcggg 1380

ggagttacga aggaaatggg cgacggcatg cacatccgtg gagatatcaa tatctgtctg 1440

atgggagatc ctggtgtggc aaagtcccag ctgctgaaat atatcgctaa ggtcgctcct 1500

cgaggcgtat acacaaccgg ccgagggagt agcggtgtcg gtctcacagc agcagtcatg 1560

agagaccctg tgaccgacga gatggttctg gagggaggag cgctggttct agcagacaat 1620

ggcatttgct gtattgacga gttcgacaaa atggaagacg gggataggac ggccatccac 1680

gaggtgatgg agcagcagac catttcgatt tcgaaggcag ggattacaac gaccctgaac 1740

gcacgtacat ccatcctggc ggccgcaaac ccgttgtatg ggcgatacaa cccacgagtg 1800

tctcccgtgg aaaacatcaa ccttcctgcg gctctactct ctcgtttcga cgtcaccttc 1860

cttgttcttg ataccccgtc acgggatgcg gacgaaaacc tggcgagtca cgtcacgtac 1920

gttcatatgc acaataagca ccccgagaac gaagacacgc cggccgtctt ctctccccac 1980

gaagtccgtc aatacatcgc caaggcgcgg acgtatcggc cggtcgtgcc gtccgcgatc 2040

tctgactaca tggttggagc atacgtgcag atgcggaagc aacagaagaa ggatgagggt 2100

agcaagagac agttttcacc agtcactcca cgtacactgc tcggtgtgat ccgtctgtcg 2160

caggcgcttg cgcgcttaag attcagtgac caggtggtca tggaggacgt ggacgaggcg 2220

ctacgtctca ttgcggttag caaggcgtcg ctcgagagcg aggaccagag cggcgttgat 2280

cacaccccga gcagcaagat ctatagtttg atccggaaca tgcgagaaag tggtgcagcg 2340

gccgtgggcg acggtgaaga cggggagctg agcatgagaa gggtcaggga gagagtgttg 2400

gccaagggat tcacggaaga ccagctgact atggcgatcg acgaatacgc caacttggat 2460

gtaagttgtt gctcggtata tgaagtctgc atttccactg acgatcaatc acaggtctgg 2520

caggtcacgg ctggagggac gcgacttgta tttatcgagg cggatggagt tgagaggatg 2580

gagatgtga 2589